# FASCICULE DE BREVET EUROPEEN

(11) **EP 0 852 492 B1**
(45) Date de publication et mention de la délivrance du brevet: **21.11.2001**
(21) Numéro de dépôt: 96932647.9
(22) Date de dépôt: 25.09.1996
(51) Int. Cl.: A61K 9/70

(54) **SYSTEME MATRICIEL TRANSDERMIQUE**
TRANSDERMALES MATRIXSYSTEM
TRANSDERMAL MATRIX SYSTEM

(30) Priorité: 27.09.1995 FR 9511326
(43) Date de publication de la demande: 15.07.1998
(73) Titulaire: LABORATOIRES D'HYGIENE ET DE DIETETIQUE, 75008 Paris (FR)
(72) Inventeur: MATH, Marie-Christine, F-21240 Talant (FR); TEILLAUD, Eric, F-21240 Talant (FR); BEVAN, Bruno, F-21800 Chevigny-Saint-Sauveur (FR)
(74) Mandataire: Hubert, Philippe
(86) Numéro de dépôt international: FR9601494
(87) Numéro de publication internationale: WO9711687

(56) Documents cités:
- EP-A- 0 093 563
- FR-A- 2 612 785
- US-A- 5 286 490
- PATENT ABSTRACTS OF JAPAN vol. 16, no. 93 (C-917), 6 Mars 1992 & JP 03 275619 A (NITSUSUI), 6 Décembre 1991,

## Description

### Domaine de l'invention

La présente invention a pour objet un nouveau système matriciel pour l'administration par voie percutanée d'un composant oestrogène et/ou un composant progestatif, ledit système étant formé d'un support et d'une matrice adhésive qui est composée d'un copolymère d'éthylène et d'acétate de vinyle (EVA) et de l'association spécifique de trois composés : le phtalate de diéthyle, le myristate de 2-octyldodécyle et une N-alkyl-2-pyrrolidone, et dans laquelle sont dissous ledit composant oestrogène et/ou ledit composant progestatif.

L'invention concerne également un procédé de préparation dudit système matriciel et son utilisation en thérapeutique.

### Art Antérieur

De nombreux systèmes d'administration par voie percutanée d'une hormone, en particulier d'un composant oestrogène seul, pour le traitement des symptômes de la ménopause et de l'ostéoporose dans le cadre de traitements qualifiés "de thérapie de remplacement des hormones déficitaires" sont aujourd'hui disponibles. Parmi ceux-ci on trouve aujourd'hui des systèmes dits "réservoirs" dans lesquels le principe actif est dissous dans un solvant servant de vecteur de transport à travers une membrane microporeuse vers la peau. Tel est le cas du dispositif à base de 17β-oestradiol commercialisé par la société CIBA-GEIGY sous la dénomination ESTRADERM® TTS.

Parallèlement il existe des systèmes dits "matriciels" dans lesquels les principes actifs sont dissous ou dispersés au sein d'une matrice adhésive à base de polymères tels des copolymères EVA, acryliques, poly(styrène-isoprène-styrène), etc. Tel est le cas du dispositif à base de 17β-oestradiol commercialisé sous la dénomination OESCLIM® par la société LABORATOIRES FOURNIER S.C.A.

En revanche, la réalisation de systèmes d'administration par voie percutanée d'un composant oestrogène et d'un composant progestatif dans ces systèmes matriciels pose encore de nombreux problèmes.

En effet, il est connu que les oestrogènes et les progestatifs sont des produits qui sont peu solubles dans les polymères utilisés dans la formulation du système matriciel. De plus, chacun de ces principes actifs peut-être partiellement ou totalement incompatible avec certains constituants de la formulation (résines, solvants, plastifiants, polymères, promoteurs d'absorption cutanée). Ils peuvent présenter des solubilités et des températures de stabilité différentes et un des deux peut recristalliser au cours du temps, se dégrader lors de la mise en oeuvre ou n'être utilisable dans la composition qu'à des concentrations trop faibles pour obtenir l'effet thérapeutique recherché. De même, il n'existe pas de promoteur d'absorption cutanée universel pour tous les principes actifs afin d'augmenter leurs flux percutanés. Donc, pour administrer des principes actifs différents, il faut souvent utiliser plusieurs promoteurs et/ ou solvants. Or, l'introduction de toute nouvelle substance pourra provoquer ou soulever de nouveaux problèmes d'irritation et de cohésion ou d'adhésion du système.

De la même manière, l'ensemble de ces contraintes (compatibilité, solubilité) joue aussi entre les différents constituants de la formulation autres que les principes actifs et augmente donc les difficultés à résoudre.

De plus à ces exigences de tolérance cutanée, d'adhésion et de cohésion du système s'ajoutent des contraintes de dosage. En général, les principes actifs présentent des perméabilités cutanées différentes qui imposent donc des flux d'absorption différents pour chaque principe actif. Il devient alors très complexe de mettre au point une formulation qui permette d'administrer la dose thérapeutique souhaitée pour chaque principe actif. En pratique, très souvent cette mise au point n'est pas réalisable et conduit à une impasse ou à des systèmes peu satisfaisants et donc économiquement non rentables. Ceci explique qu'aucun système de ce type ne soit encore commercialisé.

De fait, le seul système transdermique d'administration de deux hormones aujourd'hui disponible est un système "réservoir" à base de 17β-oestradiol et d'acétate de norethistérone, qui est commercialisé par la société CIBA-GEIGY sous la dénomination ESTRAGEST® TTS.

Il est aussi connu de l'homme de l'art que les oestrogènes et/ou les progestatifs sont des molécules qui traversent difficilement la barrière cutanée.

Ainsi, les quantités libérées de ces principes actifs pour obtenir l'effet thérapeutique recherché sont en général faibles par rapport aux quantités initiales présentes dans les dispositifs transdermiques, quel que soit leur type, ce qui a pour conséquence l'obtention de rendements faibles.

Bien que des systèmes matriciels [où la matrice est à base de copolymère d'éthylène et d'acétate de vinyle (EVA)] d'administration d'un composant oestrogène et/ou un composant progestatif aient déjà été décrits dans les demandes de brevets publiées FR-A-2 612 785, EP-A-0 279 982, WO-A-92/07589 et EP-A-055 360, aucune de ces publications ne divulgue, ni ne suggère les formulations spécifiques de l'invention qui permettent de résoudre les inconvénients décrits ci-dessus.

### Buts de l'invention

Selon l'invention on propose la réalisation de systèmes matriciels à base d'EVA pour l'administration simultanée d'un composant oestrogène et un composant progestatif pour résoudre les problèmes précités et qui présentent en plus d'excellents rendements.

On propose aussi la réalisation de tels systèmes matriciels pour l'administration d'un composant oestrogène seul ou un composant progestatif seul qui présentent d'excellents rendements.

Selon un second aspect de l'invention, on se propose de fournir un procédé de préparation de ces systèmes matriciels.

Selon encore un autre aspect de l'invention, on se propose de fournir une utilisation d'un tel système matriciel dans le traitement de la ménopause et de l'ostéoporose.

### Objet de l'invention

Les buts précités sont obtenus grâce à une nouvelle solution technique selon laquelle la matrice du système matriciel, qui contient un composant oestrogène et/ou un composant progestatif, est essentiellement constituée d'EVA et de l'association spécifique de 3 composés : le phtalate de diéthyle, le myristate de 2-octyldodécyle et une N-alkyl-2-pyrrolidone.

Plus précisément, on préconise selon l'invention un système matriciel transdermique pour l'administration par voie percutanée d'au moins une hormone, ledit système qui comprend un support et une matrice adhésive étant caractérisé en ce que ladite matrice comprend :
**(a)** 39 à 61 parties en poids de copolymère d'éthylène et d'acétate de vinyle,
**(b)** 12 à 17 parties en poids de myristate de 2-octyldodécyle,
**(c)** 5 à 17 parties en poids de phtalate de diéthyle,
**(d)** 10 à 16 parties en poids d'un composé choisi parmi les N-alkyl-2-pyrrolidones dans lesquelles le groupe alkyle est un groupe en C₄-C₁₅,
**(e)** 1 à 12 parties en poids d'au moins une hormone choisie parmi l'ensemble constitué par les composants oestrogènes et les composants progestatifs.

Selon un second aspect de l'invention on préconise aussi un système matriciel transdermique pour l'administration par voie percutanée d'au moins une hormone, ledit système qui comprend un support et une matrice adhésive étant caractérisé en ce que ladite matrice comprend :
**(a)** 39 à 61 parties en poids de copolymère d'éthylène et d'acétate de vinyle,
**(b)** 12 à 17 parties en poids de myristate de 2-octyldodécyle,
**(c)** 5 à 17 parties en poids de phtalate de diéthyle,
**(d)** 10 à 16 parties en poids d'un composé choisi parmi les N-alkyl-2-pyrrolidones dans lesquelles le groupe alkyle est un groupe en C₄-C₁₅,
**(e)** 1 à 12 parties en poids d'au moins une hormone choisie parmi l'ensemble constitué par les composants oestrogènes et les composants progestatifs, et
**(f)** 1 à 10 parties en poids d'un copolymère d'acétate de vinyle et de N-vinyl-2-pyrrolidone (en abrégé VA/VP).

Selon l'invention, on préconise également un procédé pour la préparation dudit système matriciel transdermique, ledit procédé étant caractérisé en ce qu'il comprend les étapes consistant à :
**(α)** incorporer successivement dans un réacteur à une température inférieure à la température d'ébullition du solvant ou du système de solvant utilisé, le phtalate de diéthyle, la N-alkyl-2-pyrrolidone, le myristate de 2-octyldodécyle, l'hormone choisie parmi l'ensemble constitué par les composants oestrogènes, les composants progestatifs et leurs mélanges, le copolymère VA/VP s'il est présent dans la formulation et l'EVA et agiter le mélange obtenu;
**(β)** incorporer alors le solvant ou le système de solvant et agiter l'ensemble toujours à la même température jusqu'à dissolution totale de l'EVA et homogénéisation complète dudit mélange ;
**(γ)** enduire le mélange homogène résultant de l'étape (β) sur un support temporaire antiadhérent, à une température comprise entre 50 et 70°C, pour obtenir un dépôt de 50 à 300 g/m² sur ledit support;
**(δ)** chauffer l'enduction obtenue pour évaporer le solvant ou le système de solvants à une température comprise entre 40 et 80°C, en fonction du point d'ébullition de ce dernier; et,
**(ε)** transférer la matrice sèche ainsi obtenue sur un support final.

On préconise également l'utilisation d'un système matriciel transdermique pour l'obtention d'un médicament destiné à une utilisation thérapeutique vis-à-vis des symptômes de la ménopause ou de l'ostéoporose.

### Dessins

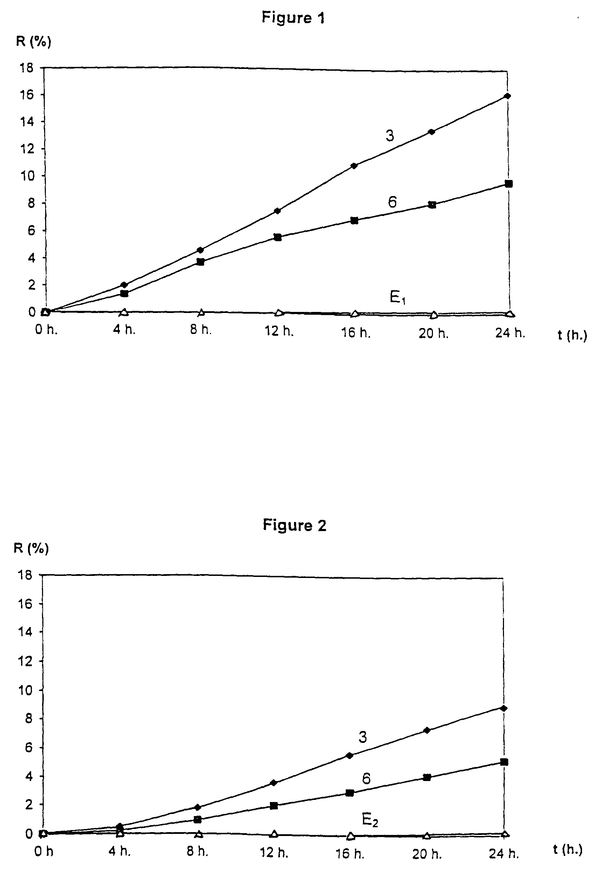

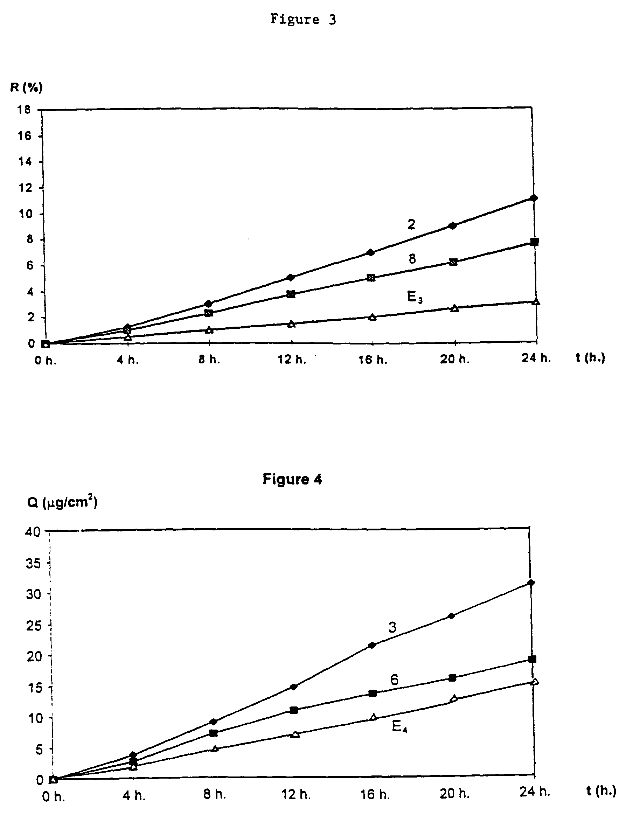

Dans les dessins annexés, la figure 4 représente la quantité (Q), exprimée en µg/cm², de 17β-oestradiol libérée en fonction du temps (t), exprimé en heures, et les figures 1 à 3 représentent le rendement (R), exprimé en %, en 17β-oestradiol ou NETA (acétate de noréthistérone) en fonction du temps (t), exprimé en heures.

Plus précisément dans ces dessins,
- **la figure 1** permet de comparer (dans le système R/t) les courbes 3, 6 et E₁ relatives au rendement de la libération de 17β-oestradiol et obtenues respectivement avec les produits des exemples 3 et 6 selon l'invention et un produit transdermique de référence connu sous la dénomination ESTRAGEST® TTS et commercialisé par la société CIBA-GEIGY (référencé ici E₁) ;
- **la figure 2** permet de comparer (dans le système R/t) les courbes 3, 6 et E₂ relatives au rendement de la libération de NETA et obtenues respectivement avec les produits des exemples 3 et 6 selon l'invention et ledit ESTRAGEST® TTS (référencé ici E₂);
- **la figure 3** permet de comparer (dans le système R/t) les courbes 2, 8 et E₃ relatives au rendement de la libération de 17β-oestradiol et obtenues respectivement avec les produits des exemples 2 et 8 selon l'invention et un produit transdermique de référence connu sous la dénomination OESCLIM® et commercialisé par la société LABORATOIRES FOURNIER S.C.A. (référencé ici E₃) ;
- **la figure 4** permet de comparer (dans le système Q/t) les courbes 3, 6 et E₄ relatives à la libération de 17β-oestradiol et obtenues respectivement avec les produits des exemples 3 et 6 selon l'invention et OESCLIM® (référencé ici E₄).

### Description détaillée de l'invention

De préférence, on utilisera un copolymère d'éthylène et d'acétate de vinyle ayant une teneur en acétate de vinyle comprise entre 30 et 75 % en poids, en particulier de l'ordre de 45 à 60 % en poids, par rapport au poids du copolymère d'éthylène/acétate de vinyle. On pourra utiliser le cas échéant un mélange de tels EVA présentant des poids moléculaires différents ou des teneurs en acétate de vinyle différentes.

Les N-alkyl-2-pyrrolidones comprennent ici les substances dans lesquelles le groupe alkyle est un groupe formé de 4 à 15 atomes de carbone, comme par exemple la N-dodécyl-2-pyrrolidone et la N-octyl-2-pyrrolidone. Dans la présente invention, on préfèrera en particulier la N-dodécyl-2-pyrrolidone. Par hormone on entend dans le cadre de la présente invention les composants oestrogènes et/ou les composants progestatifs.

Parmi les composants oestrogènes qui conviennent selon l'invention on peut citer en particulier le 17β-oestradiol et les dérivés de l'oestradiol, notamment les mono- et di-esters de l'oestradiol, comme par exemple le 17-acétate oestradiol, le 3,17-diacétate oestradiol, le 3-benzoate oestradiol, le 17-undécanoate oestradiol, les dérivés alkylés en position 17 de l'oestradiol tels que l'éthinyloestradiol, le 3-isopropylsulfonate éthinyloestradiol, le méthyloestradiol, le quinestrol, le mestranol et, le cas échéant, leurs mélanges.

Parmi les composants progestatifs qui conviennent selon l'invention on peut citer en particulier la progestérone, la médrogestérone et leurs dérivés (notamment l'acétate de 17-hydroxyprogestérone, l'acétate de médroxyprogestérone), la noréthistérone et ses dérivés (notamment l'acétate de 17-noréthistérone), le norprégnane, l'acétate de nomégestrol et le lévonorgestrel.

Selon l'invention, on utilisera de préférence comme composant oestrogène le 17β-oestradiol et comme composant progestatif l'acétate de 17-noréthistérone (NETA).

Par copolymère d'acétate de vinyle et de N-vinyl-2-pyrrolidone, on entend ici un copolymère ayant une teneur en acétate de vinyle comprise entre 30 et 70 % en poids par rapport au poids dudit copolymère. De tels produits sont bien connus pour leur utilisation comme agents filmogènes dans les aérosols et sont par exemple commercialisés sous la dénomination "PVP/VA" par la société GAF CORPORATION sous forme de poudre pour la série PVP/VA-S ou de solution dans l'éthanol ou l'isopropanol respectivement pour les séries PVP/VA-E et PVP/VA-I ou par la société BASF sous la dénomination Kollidon VA. Parmi ceux-ci, on préférera en particulier le copolymère VA/VP commercialisé sous la dénomination PVP/VA-S-630, qui contient 40 % en poids d'acétate de vinyle, et le copolymère VA/VP commercialisé sous la dénomination Kollidon VA 64 qui contient 37,7 % en poids d'acétate de vinyle.

Le support recevant la matrice pourra être tout support généralement utilisé dans les systèmes transdermiques occlusifs ou non, et imperméable aux constituants de la matrice. On préfèrera par exemple un support sous forme de film en polyéthylène, polypropylène, polyester, un complexe ou composite constitué de polyéthylène et d'un copolymère d'acétate de vinyle et d'éthylène ou des mousses.

Si nécessaire, une bande adhésive supplémentaire par exemple périphérique sous forme d'une auréole, pourra être ajoutée au système pour optimiser ses propriétés d'adhésion.

De façon pratique, la surface de la matrice qui n'est pas liée au support pourra être recouverte d'une couche ou pellicule de protection pelable avant utilisation du dispositif. Ledit dispositif pourra être lui-même emballé dans une protection étanche comme par exemple les complexes polyéthylène-aluminium.

Grâce aux excellents rendements de libération hormonale qu'il procure, le système matriciel selon l'invention présente de nombreux avantages que l'on va maintenant exposer.

Un avantage est celui du prix de revient, très nettement abaissé par rapport aux dispositifs aujourd'hui commercialisés, grâce à l'utilisation d'une plus faible quantité d'hormone(s) dont le prix est élevé.

On diminue aussi les risques de pollution de l'environnement par ces hormones lorsque le produit est jeté après la période de traitement.

Seules les formulations selon l'invention où l'on associe un copolymère EVA aux 3 composés spécifiques : N-alkyl-2-pyrrolidone, phtalate de diéthyle et myristate de 2-octyldodécyle permettent d'obtenir ces résultats.

De plus, le fait d'utiliser moins d'oestrogènes et/ou de progestatifs tout en obtenant des quantités libérées plus importantes simplifie la mise au point et la réalisation des formulations formant la matrice des dispositifs.

En effet, on minimise ou supprime ainsi les problèmes de solubilité des hormones dans les EVA ainsi que les risques d'incompatibilité chimique ou physique avec les autres constituants de la matrice. Il en est de même des problèmes de cristallisation des hormones et de l'instabilité des dispositifs au cours du temps, ces derniers phénomènes étant inacceptables pour la validation et la commercialisation de produits à visée thérapeutique tels des systèmes transdermiques.

Grâce à tous ces avantages on arrive donc enfin à obtenir un système matriciel acceptable et commercialisable pour l'administration d'un composant oestrogène et d'un composant progestatif et qui présente d'excellents rendements.

Si nécessaire, la cohésion du dispositif peut être optimisée par l'utilisation d'un mélange d'EVA de poids moléculaires différents. De même l'addition du copolymère VA/VP permet d'optimiser les propriétés d'adhésion et la solubilité des hormones dans la matrice.

De façon surprenante il permet aussi de minimiser les phénomènes d'irritation cutanée que peuvent présenter certains dispositifs.

Les systèmes transdermiques selon l'invention sont réalisés suivant les techniques généralement employées par l'homme de l'art : enduction en phase solvant ou selon la technique dite "hot-melt", c'est-à-dire en l'absence de solvant.

Dans les deux cas, dans le cadre d'une production industrielle, on enduit de grandes surfaces qui sont ensuite découpées pour donner des dispositifs aux dimensions adaptées à la dose de principe actif à administrer pendant un temps déterminé.

Dans le cadre de la technique dite "en phase solvant" on préconise un procédé pour la préparation d'un système matriciel adhésif selon l'invention qui comprend les étapes suivantes :
**(α)** on incorpore successivement dans un malaxeur, à une température inférieure à la température d'ébullition du solvant ou du système de solvant utilisé comme par exemple l'acétate d'éthyle ou un mélange acétate d'éthyle/éthanol, le phtalate de diéthyle, la N-alkyl-2-pyrrolidone, le myristate de 2-octyldodécyle, l'hormone choisie parmi l'ensemble constitué par les composants oestrogènes, les composants progestatifs et leurs mélanges, le copolymère VA/VP s'il est présent dans la formulation et l'EVA et on agite le mélange obtenu ;
**(β)** on incorpore le solvant ou le système de solvant et on agite l'ensemble toujours à la même température jusqu'à dissolution totale de l'EVA et homogénéisation complète du mélange;
**(γ)** on enduit le mélange homogène ainsi obtenu, à une température comprise entre 50 et 70°C, sur un support intermédiaire provisoire antiadhérent, notamment une pellicule de polyester siliconée à raison de 50 à 300 g/m²;
**(δ)** on évapore le solvant ou le système de solvants en chauffant à une température comprise entre 40 et 80°C, de préférence 60 à 80°C, en fonction du point d'ébullition dudit solvant ou système de solvants ; et,
**(ε)** on transfère la matrice sèche ainsi obtenue à l'étape (δ) sur le support final choisi.

Le nouveau dispositif matriciel adhésif selon l'invention est en particulier utile pour le traitement de l'ostéoporose, des symptômes de la ménopause et des risques cardio-vasculaires qui en découlent, dans le cadre d'une thérapie dite "de remplacement des hormones déficitaires" ainsi que pour tout traitement reposant sur l'administration d'oestrogènes et/ou de progestatifs par voie transdermique.

### Meilleur mode

Le meilleur mode de mise en oeuvre de l'invention consiste à faire appel à un système matriciel transdermique dont la matrice contient pour un total de 100 parties en poids :
**(a)** 54 parties en poids de copolymère d'éthylène et d'acétate de vinyle,
**(b)** 17 parties en poids de myristate de 2-octyldodécyle,
**(c)** 5 parties en poids de phtalate de diéthyle,
**(d)** 16 parties en poids de N-dodécyl-2-pyrrolidone,
**(e**_{**1**}**)** 2 parties en poids de 17β-oestradiol,
**(e**_{**2**}**)** 5 parties en poids d'acétate de noréthistérone, et
**(f)** 1 partie en poids d'un copolymère d'acétate de vinyle et de N-vinyl -2-pyrrolidone (VA/VP),
d'une part, ou
**(a)** 62 parties en poids de copolymère d'éthylène et d'acétate de vinyle,
**(b)** 13 parties en poids de myristate de 2-octyldodécyle,
**(c)** 10 parties en poids de phtalate de diéthyle,
**(d)** 10 parties en poids de N-dodécyl-2-pyrrolidone,
**(e)** 3 parties en poids de 17β-oestradiol, et,
**(f)** 2 parties en poids d'un copolymère d'acétate de vinyle et de N-vinyl -2-pyrrolidone (VA/VP),
d'autre part, ou
**(a)** 53 parties en poids de copolymère d'éthylène et d'acétate de vinyle,
**(b)** 17 parties en poids de myristate de 2-octyldodécyle,
**(c)**10 parties en poids de phtalate de diéthyle,
**(d)** 13 parties en poids de N-dodécyl-2-pyrrolidone,
**(e)** 6 parties en poids d'acétate de noréthistérone, et,
**(f)** 1 partie en poids d'un copolymère d'acétate de vinyle et de N-vinyl-2-pyrrolidone (VA/VP).

Dans ces formulations, l'EVA utilisé présente avantageusement une teneur en acétate de vinyle de 60 % en poids par rapport au poids dudit copolymère d'éthylène et d'acétate de vinyle. Le copolymère VA/VP présente lui avantageusement une teneur en acétate de vinyle de 35 à 40 % en poids par rapport au poids dudit copolymère VA/VP.

D'autres avantages et caractéristiques de l'invention seront mieux compris à la lecture de la description qui va suivre d'exemples de réalisation et d'essais comparatifs.

Bien entendu l'ensemble de ces éléments n'est nullement limitatif mais est donné à titre d'illustration.

Par commodité, dans ce qui suit les abréviations suivantes ont été utilisées :
**EVA :** copolymère d'éthylène et d'acétate de vinyle
**Es :** 17β-oestradiol
**NETA :** acétate de noréthistérone
**VA/VP :** copolymère d'acétate de vinyle et de N-vinyl-2-pyrrolidone.

### Exemple 1

Dans un bécher de 250 ml, on introduit successivement 0,62 g de 17β-oestradiol, 1,2 g de NETA, 3 g de SURFADONE® LP 300 (N-dodécyl-2-pyrrolidone commercialisée par la société GAF CORPORATION), 3,9 g de myristate de 2-octyldodécyle commercialisé par la société GATTEFOSSE (en abrégé ci-après : "M.O.D."), 3 g de phtalate de diéthyle et 40,5 g d'acétate d'éthyle. On chauffe le mélange obtenu à une température comprise entre 65 et 75°C tout en agitant. On introduit alors par fractions 18,3 g de LEVAPREN® 600 HV (copolymère EVA ayant une teneur de 60 % en poids en motifs acétate de vinyle et commercialisé par la société BAYER) et on agite le mélange obtenu en chauffant toujours entre 65 et 75°C pendant environ 50 minutes jusqu'à dissolution complète du copolymère EVA. On réajuste l'extrait sec à 50 % en poids et on laisse dégazer le mélange obtenu. On enduit ce dernier de manière à former un dépôt de (100±10) g/m² sur un support temporaire en polyester siliconé à température comprise entre 65 et 75°C. On chauffe l'enduction ainsi obtenue à 70°C pendant au moins 15 minutes afin d'évaporer le solvant. On transfère alors la matrice ainsi obtenue sur un support final en polyester. On découpe à la taille désirée et conditionne les produits ainsi découpés dans des sachets thermosoudables.

### Exemple 2

On procède de façon analogue à l'exemple 1, mais on utilise dans ce cas 0,83 g de Es, 1,6 g de NETA, 4 g de SURFADONE® LP 300, 6 g de M.O.D, 6 g de phtalate de diéthyle, 54 g d'acétate d'éthyle et 21,6 g de LEVAPREN® 600 HV (copolymère EVA ayant une teneur de 60 % en poids en motifs acétate de vinyle et commercialisé par la société BAYER).

### Exemple 3

On procède de façon analogue à l'exemple 1, mais dans ce cas on utilise 0,62 g de Es, 1,2 g de NETA, 4,8 g de SURFADONE® LP 300, 5,1 g de M.O.D., 3 g de phtalate de diéthyle, 40,5 g d'acétate d'éthyle et 15,3 g de LEVAPREN® 600 HV.

### Exemple 4

On procède de façon analogue à l'exemple 1, mais on utilise dans ce cas un mélange de copolymères EVA. On incorpore 0,62 g de Es, 1,5 g de NETA, 3 g de SURFADONE® LP 300, 4,5 g de M.O.D, 3 g de phtalate de diéthyle, 40,5 g d'acétate d'éthyle et un mélange de 14,4 g de LEVAPREN® 600 HV et 3 g de LEVAPREN® 400 (copolymère EVA ayant une teneur de 40 % en poids en motifs acétate de vinyle et commercialisé par la société BAYER).

### Exemple 5

Dans un réacteur on incorpore à température ambiante 201,6 g de phtalate de diéthyle, 101,3 g de SURFADONE® LP 300, 100,8 g de M.O.D, 20,7 g de Es, 40 g de NETA et 539,9 g de LEVAPREN® 500 HV (copolymère EVA ayant une teneur de 50 % en poids en motifs acétate de vinyle, et commercialisé par la société BAYER) et on agite l'ensemble. On incorpore alors 1003,7 g d'acétate d'éthyle et on chauffe à environ 75°C sous agitation jusqu'à dissolution complète du copolymère EVA. On laisse dégazer le mélange obtenu. On enduit ce dernier de manière à former un dépôt de (100±10) g/m², à une température de 50°C sur un support temporaire en polyester siliconé. On place alors le produit enduit dans un tunnel de séchage dont la température varie de 60 à 80°C afin d'évaporer le solvant et on transfère la matrice ainsi obtenue sur un support final en polyester. On découpe à la taille désirée et conditionne les produits ainsi découpés dans des sachets thermosoudables.

### Exemple 6

Dans un réacteur, on incorpore à 75°C 130,5 g de M.O.D, 130,9 g de phtalate de diéthyle, 100,9 g de SURFADONE® LP 300, 20,7 g de Es, 40 g de NETA, 570,3 g de LEVAPREN® 600 HV, 10,1 g de PVP/VA-S-630 (copolymère d'acétate de vinyle et de N-vinyl-2-pyrrolidone contenant 40 % en poids d'acétate de vinyle commercialisé par la société GAF CORPORATION) et on agite le tout pendant 5 minutes. On incorpore alors 989,9 g d'acétate d'éthyle et 10,1 g d'éthanol. On agite l'ensemble toujours à 75°C jusqu'à dissolution complète du copolymère EVA et on laisse dégazer le mélange obtenu. On enduit ce dernier à une température de 50°C de façon à former un dépôt de (100±10) g/m² sur un support temporaire en polyester siliconé. On place alors le produit enduit dans un tunnel de séchage dont la température varie de 60 à 80°C afin d'évaporer les solvants et on transfère la matrice ainsi obtenue sur un support final en polyester. On découpe à la taille désirée et on conditionne les produits ainsi découpés dans des sachets thermosoudables.

### Exemple 7

Dans un réacteur, on incorpore à 75°C 201,4 g de phtalate de diéthyle, 201,1 g de SURFADONE® LP 300, 340,1 g de M.O.D, 40 g de Es, 80 g de NETA, 60 g de PVP/VA-S-630 et 1080,2 g de LEVAPREN® 500 HV et on agite le mélange obtenu. On incorpore alors 1999,8 g d'acétate d'éthyle et agite le mélange toujours à environ 75°C pendant au minimum 4 heures jusqu'à dissolution complète du copolymère EVA. On laisse dégazer le mélange obtenu. On enduit ce dernier à une température de 50°C de façon à former un dépôt de (100±10) g/m² sur un support temporaire en polyester siliconé. On place alors le produit enduit dans un tunnel de séchage dont la température varie de 60 à 80°C afin d'évaporer les solvants et on transfère la matrice ainsi obtenue sur un support final en polyester. On découpe à la taille désirée et on conditionne les produits ainsi découpés dans des sachets thermosoudables.

### Exemple 8

Dans un bécher de 250 ml on introduit 0,62 g de Es, 1,5 g de NETA, 3 g de SURFADONE® LP 300, 4,5 g de M.O.D, 3 g de phtalate de diéthyle, 0,3 g de PVP/VA-S-630, 10 g d'éthanol et 40,5 g d'acétate d'éthyle. On chauffe le mélange obtenu, tout en agitant, à une température comprise entre 65 et 75°C jusqu'à dissolution complète du copolymère VA/VP. On introduit alors, par fractions, 17,1 g de LEVAPREN® 600 HV et on agite le mélange obtenu toujours en chauffant entre 65 et 75°C pendant environ 50 minutes jusqu'à dissolution complète du copolymère EVA. On réajuste l'extrait sec à 50 % en poids et on laisse dégazer le mélange obtenu. On enduit ce dernier de manière à former un dépôt de (100±10) g/m² sur un support temporaire en polyester siliconé à une température comprise entre 65 et 75°C. On chauffe l'enduction ainsi obtenue à 70°C pendant au minimum 15 minutes afin d'évaporer les solvants et on transfère la matrice ainsi obtenue sur un support final en polyester. On découpe à la taille désirée et conditionne les produits ainsi découpés dans des sachets thermosoudables.

### Exemple 9

On procède de façon analogue à l'exemple 1, mais on incorpore dans ce cas une seule hormone : le NETA. On utilise donc ici 1,2 g de NETA, 3 g de SURFADONE® LP 300, 7,5 g de M.O.D, 1,5 g de phtalate de diéthyle 40,5 g d'acétate d'éthyle et 16,8 g de LEVAPREN® 500 HV.

### Exemple 10

On procède de façon analogue à l'exemple 9, mais on utilise dans ce cas 4,8 g de NETA, 19,2 g de SURFADONE® LP 300, 20,4 g de M.O.D, 6 g de phtalate de diéthyle, 162 g d'acétate d'éthyle et 69,6 g de LEVAPREN® 600 HV.

### Exemple 11

Dans un bécher de 250 ml, on introduit 1,8 g de NETA, 4,8 g de SURFADONE® LP 300, 5,1 g de M.O.D, 3 g de phtalate de diéthyle et 40,5 g d'acétate d'éthyle et on chauffe l'ensemble à environ 75°C en agitant. On introduit alors, par petites fractions, 12 g de LEVAPREN® 600 HV et 3 g de LEVAPREN® 400 et on agite toujours à 75°C pendant environ une heure jusqu'à dissolution complète du copolymère EVA. On réajuste l'extrait sec à 50 % en poids et on introduit 0,6 g de PVP/VA-S 630 en solution éthanolique à 50 % en poids. On agite le tout et on laisse dégazer le mélange obtenu. On enduit ce dernier de manière à former un dépôt de 100±10 g/m² sur un support temporaire en polyester siliconé à une température comprise entre 65 et 75°C. On chauffe l'enduction ainsi obtenue à 70°C pendant au moins 15 minutes afin d'évaporer les solvants. On transfère alors la matrice ainsi obtenue sur un support final en polyester. On découpe à la taille désirée et on conditionne les produits ainsi découpés dans des sachets thermosoudables.

### Exemple 12

Dans un bécher de 250 ml, on introduit 0,93 g de Es, 3 g de SURFADONE® LP 300, 3,9 g de M.O.D, 3 g de phtalate de diéthyle, 0,6 g de KOLLIDON VA-64 (copolymère d'acétate de vinyle et de N-vinyl-2-pyrrolidone ayant 37,7 % en poids d'acétate de vinyle commercialisé par la société BASF), 37,5 g d'acétate d'éthyle et 3 g d'éthanol. On chauffe l'ensemble à 75°C sous agitation jusqu'à dissolution complète du KOLLIDON VA-64. On introduit alors, par petites fractions, 18,6 g de LEVAPREN® 600 HV et on agite pendant environ 1 heure jusqu'à dissolution complète du copolymère EVA. On réajuste l'extrait sec à 50 % en poids et on laisse dégazer le mélange obtenu. On enduit ce dernier de manière à former un dépôt de (100±10) g/m² sur un support temporaire en polyester siliconé à une température comprise entre 65 et 75°C. On chauffe ensuite l'enduction, ainsi obtenue, à 70°C pendant au moins 15 minutes afin d'évaporer les solvants. On transfère alors la matrice, ainsi obtenue, sur un support final en polyester. On découpe à la taille désirée et on conditionne les produits ainsi découpés dans des sachets thermosoudables.

### Exemple 13

On procède de façon analogue à l'exemple 12, mais on incorpore dans ce cas une seconde hormone : le NETA. On utilise donc ici 1,5 g de NETA, 0,62 g de Es, 4,8 g de SURFADONE® LP 300, 5,1 g de M.O.D, 1,5 g de phtalate de diéthyle, 0,3 g de KOLLIDON VA-64, 37,5 g d'acétate d'éthyle, 3 g d'éthanol et 16,2 g de LEVAPREN® 600.

### Exemple 14

On procède de façon analogue à l'exemple 11, mais on emploie un seul solvant, l'acétate d'éthyle; on utilise dans ce cas 1,8 g de NETA, 3,9 g de SURFADONE® LP 300, 5,1 g de M.O.D, 3 g de phtalate de diéthyle, 40,5 g d'acétate d'éthyle, 15,9 g de LEVAPREN® 600 HV et 0,6 g de PVP/VA-S-630.

### Essais

Les rendements des dispositifs selon l'invention sont déterminés à partir des mesures de quantités d'hormone(s) libérée(s) en 24 heures sur un modèle de peau ex-vivo.

Pour cela on a réalisé des tests de perméation ex-vivo sur peau abdominale de souris "nude" mâle selon le protocole suivant.

La mesure des quantités d'hormone(s) libérée(s) par un dispositif transdermique d'une surface de 2,54 cm², préalablement découpé à l'emporte-pièce et déposé sur un disque de 3,14 cm² de peau abdominale de souris "nude" mâle, est effectuée dans une cellule statique en verre thermostatée à 37°C et ayant un compartiment récepteur d'un volume de 11,5 ml, ce compartiment récepteur contenant comme phase réceptrice un mélange sérum physiologique/PEG 400 (75/25 v/v).

Compte-tenu de la variabilité des résultats liés à la perméabilité intrinsèque des échantillons de peau, chaque essai de perméation pour un échantillon de dispositif transdermique est réalisé sur un nombre minimum de 3 à 5 échantillons de peau.

Le résultat qui est donné est la moyenne obtenue pour chaque dispositif, à partir de ces essais. Le rapport entre cette valeur moyenne des quantités d'hormone(s) libérée(s) en fin de cinétique en 24 heures et la quantité initiale d'hormone(s) contenue dans le dispositif permet d'évaluer le rendement en 24 heures des systèmes transdermiques selon l'invention.

Dans un but comparatif on a déterminé de la même façon les quantités d'hormones libérées à 24 heures du seul produit aujourd'hui disponible comprenant à la fois un oestrogène et un progestatif, à savoir le dispositif commercialisé sous la marque ESTRAGEST® TTS par la société CIBA-GEIGY. C'est d'ailleurs aussi le seul système transdermique commercial qui contienne un composant progestatif.

Le dispositif ESTRAGEST® TTS est formé de deux réservoirs accolés contenant au total 10 mg de 17β-oestradiol et 30 mg de NETA, chaque réservoir contenant un mélange de 5 mg de 17β-oestradiol et 15 mg de NETA.

Les mesures de perméation cutanée sont effectuées suivant le même protocole sur un seul des deux réservoirs placé sur un échantillon de peau de 3,14 cm². Les quantités initiales d'hormones contenues dans ce réservoir sont ramenées à la quantité initiale d'hormones par unité de surface exprimée en µg/cm².

Le rapport de la valeur moyenne des quantités de 17β-oestradiol ou de NETA libérées en 24 heures à la quantité initiale contenue dans le réservoir permet d'obtenir les rendements à 24 heures en Es ou en NETA.

Les résultats obtenus ont été consignés dans le tableau I pour les dispositifs selon l'invention contenant un oestrogène et un progestatif et dans le tableau II pour les dispositifs contenant seulement un progestatif.

On a aussi comparé les rendements obtenus en 24 heures par un dispositif selon l'invention pour le 17β-oestradiol par rapport au seul dispositif matriciel à base de copolymère EVA aujourd'hui commercialisé, à savoir le produit OESCLIM® . Pour cela on a effectué, toujours selon le même protocole, des tests de perméation ex-vivo sur peau abdominale de souris "nude" mâle à l'aide d'échantillons de 2,54 cm² de OESCLIM® . Avec ce dispositif matriciel, selon plusieurs séries de tests on trouve en moyenne une valeur initiale de 17β-oestradiol par unité de surface de 452,7 µg/cm² et une quantité de 17β-oestradiol libérée au bout de 24 heures sur ce modèle de peau de 14,2 µg/cm²

Le tableau III présente les rendements calculés pour les dispositifs selon l'invention des exemples 1, 2, 3, 6 et 12, en ne tenant compte que du 17β-oestradiol, et pour le produit OESCLIM® .

Dans le cas d'une matrice contenant à la fois du 17β-oestradiol et du NETA, le tableau I illustre les avantages des systèmes selon l'invention vis à vis du produit ESTRAGEST® TTS précité. On constate dans ce cas, comme le montrent les courbes 3 et 6 des figures 1 et 2, que l'on a toujours des rendements pour les dispositifs selon l'invention pour le 17β-oestradiol comme pour le NETA qui sont significativement supérieurs à ceux du système ESTRAGEST® TTS, ceci avec des quantités initiales moindres, respectivement 8 et 12 fois inférieures.

De plus, comme le montre le tableau I, dans le cas du 17β-oestradiol, on a des rendements en moyenne 25 à 80 fois supérieurs à celui d' ESTRAGEST® TTS, et dans le cas du NETA des rendements 20 à 50 fois supérieurs à celui d' ESTRAGEST® TTS.

Plus précisément, on a par rapport à ESTRAGEST® TTS :
- pour l'exemple 1 un rendement 47 fois supérieur pour Es et 29 fois supérieur pour NETA,
- pour l'exemple 2 un rendement 55 fois supérieur pour Es et 32 fois supérieur pour NETA,
- pour l'exemple 3 un rendement 81 fois supérieur pour Es et 52 fois supérieur pour NETA,
- pour l'exemple 4 un rendement 36 fois supérieur pour Es et 29 fois supérieur pour NETA,
- pour l'exemple 5 un rendement 26 fois supérieur pour Es et 18 fois supérieur pour NETA,
- pour l'exemple 6 un rendement 48 fois supérieur pour Es et 31 fois supérieur pour NETA,
- pour l'exemple 7 un rendement 29 fois supérieur pour Es et 18 fois supérieur pour NETA,
- pour l'exemple 8 un rendement 38 fois supérieur pour Es et 23 fois supérieur pour NETA.
- pour l'exemple 13 un rendement 75 fois supérieur pour Es et 50 fois supérieur pour NETA.

Ces différences importantes démontrent à nouveau les avantages de l'invention, à savoir de permettre de réaliser des gains de coûts importants en utilisant moins de produit dans le but thérapeutique recherché, d'éviter les problèmes éventuels de cristallisation et d'incompatibilité dans la matrice, donc de simplifier la mise au point des systèmes et de fabriquer en particulier des systèmes matriciels d'administration d'un composant oestrogène et d'un composant progestatif performants et acceptables commercialement.

Le copolymère VA/VP, qui intervient dans les exemples 6-8 et 13, est suivant le cas, utile pour améliorer l'adhérence des dispositifs, la solubilité des hormones dans la matrice ou de façon surprenante les phénomènes d'irritation cutanée qui peuvent apparaître à l'usage. Selon l'invention, eu égard aux quantités utilisées, la présence du copolymère VA/VP ne gène pas l'obtention de bons rendements.

De même dans le cas du mélange de deux EVA ayant des teneurs en acétate de vinyle différentes (exemple 4) on a toujours d'aussi bons résultats.

De façon similaire, on a des résultats analogues dans le cas de systèmes contenant Es seul ou NETA seul comme cela est illustré par les figures 3 et 4, d'une part, et les tableaux II et III, d'autre part.

Les résultats du tableau II montrent que l'on a toujours des quantités libérées de NETA supérieures à celle du dispositif ESTRAGEST® TTS et ceci malgré les concentrations 12 fois inférieures.

L'analyse des rendements du tableau II montre que, par rapport au dispositif ESTRAGEST® TTS, on a respectivement des rendements pour NETA 26 fois, 39 fois et 25 fois et 48 fois supérieurs pour les produits des exemples 9, 10, 11 et 14.

Les résultats du tableau III mettent également en évidence les avantages des dispositifs selon l'invention par rapport au produit OESCLIM® dans le cas du 17β-oestradiol. Par rapport au produit OESCLIM® , qui est le seul système matriciel à base d'EVA aujourd'hui commercialisé, on constate à nouveau que les rendements des dispositifs selon l'invention que ce soit pour les exemples 1, 2, 3 et 6 qui contiennent du 17β-oestradiol et NETA ou pour l'exemple 12 qui contient comme OESCLIM® seulement du 17β-oestradiol sont toujours supérieurs de 3 à 5 fois dans le meilleur des cas. Ce résultat est aussi illustré par les courbes 2 et 8 (relatives aux exemples 2 et 8) de la figure 3.

De même, les courbes 3 et 6 de la figure 4 montrent que l'on a toujours des quantités de 17β-oestradiol libérées par les dispositifs selon l'invention qui sont significativement supérieures à celle du produit OESCLIM® ceci avec des quantités 2,3 fois inférieures.

Les conclusions tirées du tableau I, dans le cas de l'administration de deux hormones, sur les avantages des dispositifs selon l'invention sont donc dans le cas de l'administration d'une seule hormone à nouveau évidentes et identiques.

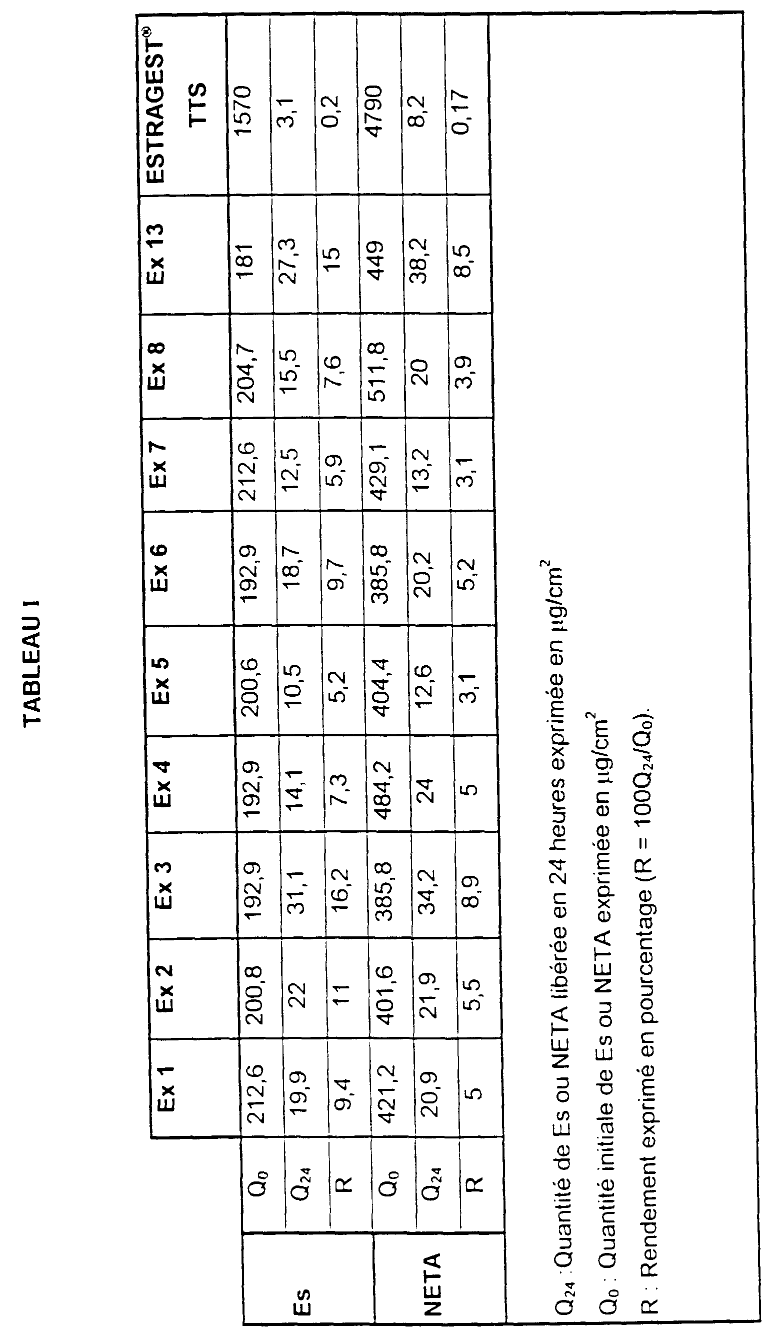

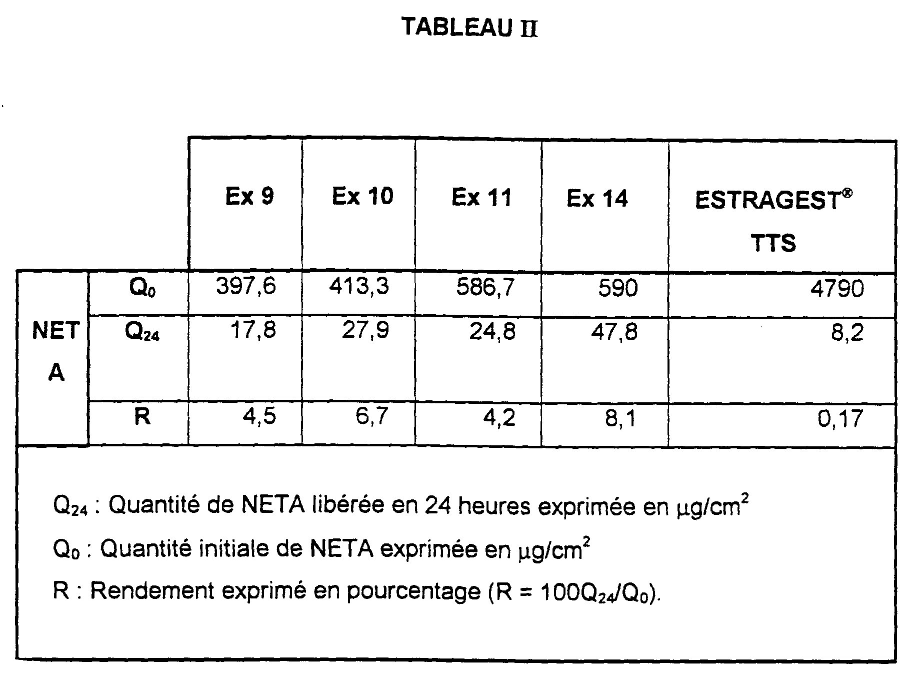

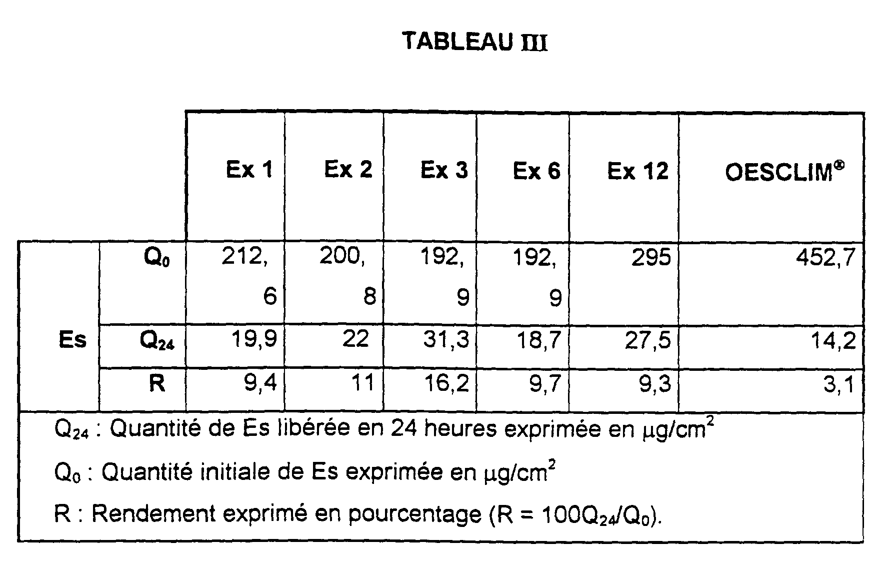

## Revendications

1. Système matriciel transdermique pour l'administration par voie percutanée d'au moins une hormone , ledit système, qui comprend un support et une matrice adhésive, étant **caractérisé en ce que** ladite matrice comprend:
**(a)** 39 à 61 parties en poids de copolymère d'éthylène et d'acétate de vinyle (EVA),
**(b)** 12 à 17 parties en poids de myristate de 2-octyldodécyle,
**(c)** 5 à 17 parties en poids de phtalate de diéthyle,
**(d)** 10 à 16 parties en poids d'un composé choisi parmi les N-alkyl-2-pyrrolidones, dans lesquelles le groupe alkyle est un groupe en C₄-C₁₅,
**(e)** 1 à 12 parties en poids d'au moins une hormone choisie parmi l'ensemble constitué par les composants oestrogènes et les composants progestatifs.

2. Système matriciel transdermique selon la revendication 1, **caractérisé en ce que** la matrice adhésive comprend en outre 1 à 10 parties en poids d'un copolymère d'acétate de vinyle et de N-vinyl-2-pyrrolidone (VA/VP).

3. Système matriciel transdermique selon la revendication 2, **caractérisé en ce que** le copolymère d'acétate de vinyle et de N-vinyl-2-pyrrolidone a une teneur en acétate de vinyle comprise entre 30 et 70 % en poids par rapport au poids dudit copolymère.

4. Système matriciel transdermique selon l'une quelconque des revendications 1 à 3, **caractérisé en ce que** la N-alkyl-2-pyrrolidone est la N-dodécyl-2-pyrrolidone.

5. Système matriciel transdermique selon l'une quelconque des revendications 1 à 3, **caractérisé en ce que** le copolymère d'éthylène et d'acétate de vinyle a une teneur en acétate de vinyle comprise entre 30 et 75 % en poids par rapport au poids dudit copolymère, de préférence 60 %.

6. Système matriciel transdermique selon l'une quelconque des revendications 1 à 3, **caractérisé en ce que** l'hormone est un composant oestrogène, de préférence le 17β-oestradiol.

7. Système matriciel transdermique selon l'une quelconque des revendications 1 à 3, **caractérisé en ce que** l'hormone est un composant progestatif, de préférence l'acétate de noréthistérone.

8. Système matriciel transdermique selon l'une quelconque des revendications 1 à 3, **caractérisé en ce que** le système contient un mélange constitué d'un composant oestrogène et d'un composant progestatif, de préférence un mélange de 17β-oestradiol et d'acétate de noréthistérone.

9. Procédé pour la préparation d'un système matriciel transdermique suivant l'une quelconque des revendications 1 à 8, ledit procédé étant **caractérisé en ce qu'**il comprend les étapes consistant à :
**(α)** incorporer successivement dans un réacteur à une température inférieure à la température d'ébullition du solvant ou du système de solvants utilisé, le phtalate de diéthyle, la N-alkyl-2-pyrrolidone, le myristate de 2-octyldodécyle, au moins une hormone choisie parmi l'ensemble constitué par les composants oestrogènes, les composants progestatifs et leurs mélanges, le copolymère VA/VP s'il est présent dans la formulation et l'EVA, et agiter le mélange obtenu ;
**(β)** incorporer alors le solvant ou le système de solvants et agiter l'ensemble toujours à la même température jusqu'à dissolution totale de l'EVA et homogénéisation complète dudit mélange ;
**(γ)** enduire le mélange homogène résultant de l'étape (β) sur un support temporaire antiadhérent, à une température comprise entre 50 et 70°C, pour obtenir un dépôt de 50 à 300 g/m² sur ledit support ;
**(δ)** chauffer l'enduction obtenue pour évaporer le solvant ou le système de solvants à une température comprise entre 40 et 80°C, en fonction du point d'ébullition de ce dernier ; et,
**(ε)** transférer la matrice sèche ainsi obtenue sur un support final.

10. Utilisation d'un système matriciel transdermique suivant l'une quelconque des revendications 1 à 8 pour l'obtention d'un médicament destiné à une utilisation thérapeutique vis-à-vis des symptômes de la ménopause ou de l'ostéoporose.

11. Système selon la revendication 2, **caractérisé en ce que** sa matrice comprend, pour un total de 100 parties en poids :
**(a)** 54 parties en poids de copolymère d'éthylène et d'acétate de vinyle,
**(b)** 17 parties en poids de myristate de 2-octyldodécyle,
**(c)** 5 parties en poids de phtalate de diéthyle,
**(d)** 16 parties en poids de N-dodécyl-2-pyrrolidone,
**(e**_{**1**}**)** 2 parties en poids de 17β-oestradiol,
**(e**_{**2**}**)** 5 parties en poids d'acétate de noréthistérone, et
**(f)** 1 partie en poids d'un copolymère d'acétate de vinyle et de N-vinyl-2-pyrrolidone (VA/VP).

12. Système selon la revendication 2, **caractérisé en ce que** sa matrice comprend, pour un total de 100 parties en poids :
**(a)** 62 parties en poids de copolymère d'éthylène et d'acétate de vinyle,
**(b)** 13 parties en poids de myristate de 2-octyldodécyle,
**(c)** 10 parties en poids de phtalate de diéthyle,
**(d)** 10 parties en poids de N-dodécyl-2-pyrrolidone,
**(e)** 3 parties en poids de 17β-oestradiol, et,
**(f)** 2 parties en poids d'un copolymère d'acétate de vinyle et de N-vinyl-2-pyrrolidone (VA/VP).

13. Système selon la revendication 2, **caractérisé en ce que** sa matrice comprend, pour un total de 100 parties en poids :
**(a)** 53 parties en poids de copolymère d'éthylène et d'acétate de vinyle,
**(b)** 17 parties en poids de myristate de 2-octyldodécyle,
**(c)** 10 parties en poids de phtalate de diéthyle,
**(d)** 13 parties en poids de N-dodécyl-2-pyrrolidone,
**(e)** 6 parties en poids d'acétate de noréthistérone, et,
**(f)** 1 partie en poids d'un copolymère d'acétate de vinyle et de N-vinyl-2-pyrrolidone (VA/VP).

## Patentansprüche

1. Transdermales Matrixsystem zur perkutanen Verabreichung mindestens eines Hormons, wobei dieses System, das einen Träger und eine Haftmatrix umfasst, **dadurch gekennzeichnet ist, dass** diese Matrix
a) 39 bis 61 Gew.-Teile eines Ethylen-Vinylacetat-Copolymers (EVA),
b) 12 bis 17 Gew.-Teile 2-Octyldodecylmyristat,
c) 5 bis 17 Gew.-Teile Diethylphthalat,
d) 10 bis 16 Gew.-Teile einer Verbindung, die aus den N-Alkyl-2-pyrrolidonen, deren Alkylgruppe eine C₄- bis C₁₅-Gruppe ist, ausgewählt ist, und
e) 1 bis 12 Gew.-Teile mindestens eines Hormons, das aus der Gesamtheit, die von östrogenen Komponenten und Gestagen-Komponenten gebildet wird, ausgewählt ist,
enthält.

2. Transdermales Matrixsystem nach Anspruch 1, **dadurch gekennzeichnet, dass** die Haftmatrix außerdem 1 bis 10 Gew.-Teile eines Vinylacetat-N-Vinyl-2-pyrrolidon-Copolymers (VA/VP) enthält.

3. Transdermales Matrixsystem nach Anspruch 2, **dadurch gekennzeichnet, dass** das Vinylacetat-N-Vinyl-2-pyrrolidon-Copolymer einen Vinylacetatanteil von 30 bis 70 Gew.-% des Copolymergewichts besitzt.

4. Transdermales Matrixsystem nach einem der Ansprüche 1 bis 3, **dadurch gekennzeichnet, dass** das N-Alkyl-2-pyrrolidon N-Dodecyl-2-pyrrolidon ist.

5. Transdermales Matrixsystem nach einem der Ansprüche 1 bis 3, **dadurch gekennzeichnet, dass** das Ethylen-Vinylacetat-Copolymer einen Vinylacetatanteil von 30 bis 75 Gew.-% und vorzugsweise 60 Gew.-% des Copolymergewichts besitzt.

6. Transdermales Matrixsystem nach einem der Ansprüche 1 bis 3, **dadurch gekennzeichnet, dass** das Hormon eine östrogene Komponente und vorzugsweise 17β-Östradiol ist.

7. Transdermales Matrixsystem nach einem der Ansprüche 1 bis 3, **dadurch gekennzeichnet, dass** das Hormon eine Gestagen-Komponente und vorzugsweise Norethisteronacetat ist.

8. Transdermales Matrixsystem nach einem der Ansprüche 1 bis 3, **dadurch gekennzeichnet, dass** das System ein Gemisch, das aus einer östrogenen Komponente und einer Gestagen-Komponente besteht, und vorzugsweise ein Gemisch aus 17β-Östradiol und Norethisteronacetat enthält.

9. Verfahren zur Herstellung eines transdermalen Matrixsystems nach einem der Ansprüche 1 bis 8, wobei das Verfahren **dadurch gekennzeichnet ist, dass** es die Stufen, die aus
α) nacheinander Einfüllen von Diethylphthalat, N-Alkyl-2-pyrrolidon, 2-Octyldodecylmyristat, mindestens einem Hormon, das aus der Gesamtheit ausgewählt ist, die aus östrogenen Komponenten, Gestagen-Komponenten und ihren Gemischen besteht, VA/VP-Copolymer, falls es in der Formulierung vorhanden ist, und EVA in einen Reaktor bei einer Temperatur unterhalb der Siedetemperatur des verwendeten Lösungsmittels oder Lösungsmittelsystems und Rühren des erhaltenen Gemischs,
β) Zugeben des Lösungsmittels oder Lösungsmittelsystems und Rühren des Ganzen bei derselben Temperatur wie zuvor bis zur vollständigen Auflösung des EVA und zur vollkommenen Homogenisierung des Gemischs,
γ) Auftragen des aus Stufe β) resultierenden homogenen Gemischs bei einer Temperatur von 50 bis 70 °C auf einen nicht haftenden temporären Träger, um darauf eine Schicht von 50 bis 300 g/m² zu erhalten,
δ) Erhitzen der erhaltenen Schicht bei einer Temperatur von 40 bis 80 °C, um das Lösungsmittel oder Lösungsmittelsystem in Abhängigkeit von dessen Siedepunkt zu verdampfen, und
ε) Übertragen der so erhaltenen getrockneten Matrix auf einen endgültigen Träger
bestehen, umfasst.

10. Verwendung eines transdermalen Matrixsystems nach einem der Ansprüche 1 bis 8 zur Herstellung eines Arzneimittels, das für eine therapeutische Anwendung bei Symptomen der Menopause oder Osteoporose vorgesehen ist.

11. System nach Anspruch 2, **dadurch gekennzeichnet, dass** seine Matrix
a) 54 Gew.-Teile Ethylen-Vinylacetat-Copolymer,
b) 17 Gew.-Teile 2-Octyldodecylmyristat,
c) 5 Gew.-Teile Diethylphthalat,
d) 16 Gew.-Teile N-Dodecyl-2-pyrrolidon,
e₁) 2 Gew.-Teile 17β-Östradiol,
e2) 5 Gew.-Teile Norethisteronacetat und
f) 1 Gew.-Teil eines Vinylacetat-N-Vinyl-2-pyrrolidon-Copolymers (VA/VP)
auf insgesamt 100 Gew.-Teile enthält.

12. System nach Anspruch 2, **dadurch gekennzeichnet, dass** seine Matrix
a) 62 Gew.-Teile Ethylen-Vinylacetat-Copolymer,
b) 13 Gew.-Teile 2-Octyldodecylmyristat,
c) 10 Gew.-Teile Diethylphthalat,
d) 10 Gew.-Teile N-Dodecyl-2-pyrrolidon,
e) 3 Gew.-Teile 17β-Östradiol und
f) 2 Gew.-Teile eines Vinylacetat-N-Vinyl-2-pyrrolidon-Copolymers (VA/VP)
auf insgesamt 100 Gew.-Teile enthält.

13. System nach Anspruch 2, **dadurch gekennzeichnet, dass** seine Matrix
a) 53 Gew.-Teile Ethylen-Vinylacetat-Copolymer,
b) 17 Gew.-Teile 2-Octyldodecylmyristat,
c) 10 Gew.-Teile Diethylphthalat,
d) 13 Gew.-Teile N-Dodecyl-2-pyrrolidon,
e) 6 Gew.-Teile Norethisteronacetat und
f) 1 Gew.-Teil eines Vinylacetat-N-Vinyl-2-pyrrolidon-Copolymers (VA/VP)
auf insgesamt 100 Gew.-Teile enthält.

## Claims

1. A transdermal matrix system for the transdermal administration of at least one hormone, said system, which comprises a carrier and an adhesive matrix, being **characterised in that** said matrix comprises:
(a) 39 to 61 parts by weight of ethylene/vinyl acetate (EVA) copolymer,
(b) 12 to 17 parts by weight of 2-octyldodecyl myristate,
(c) 5 to 17 parts by weight of diethyl phthalate,
(d) 10 to 16 parts by weight of a compound selected from N-alkyl-2-pyrrolidones in which the alkyl group is a C₄ -C₁₅ group, and
(e) 1 to 12 parts by weight of at least one hormone selected from the group consisting of estrogen components and progestin components.

2. A transdermal matrix system according to claim 1 wherein said adhesive matrix also comprises 1 to 10 parts by weight of a vinyl acetate/N-vinyl-2-pyrrolidone (VA/VP) copolymer.

3. A transdermal matrix system according to claim 2 wherein the vinyl acetate/N-vinyl-2-pyrrolidone copolymer has a vinyl acetate content of between 30 and 70% by weight, based on the weight of the copolymer.

4. A transdermal matrix system according to any one of claims 1 to 3 wherein the N-alkyl-2-pyrrolidone is N-dodecyl-2-pyrrolidone.

5. A transdermal matrix system according to any one of claims 1 to 3 wherein the ethylene/vinyl acetate copolymer has a vinyl acetate content of between 30 and 75% by weight, preferably 60%, based on the weight of the copolymer.

6. A transdermal matrix system according to any one of claims 1 to 3 wherein the hormone is an estrogen component, preferably 17 β-estradiol.

7. A transdermal matrix system according to any one of claims 1 to 3 wherein the hormone is a progestin component, preferably norethisterone acetate.

8. A transdermal matrix system according to any one of claims 1 to 3 wherein the system contains a mixture of an estrogen component and a progestin component, preferably a mixture of 17 β-estradiol and norethisterone acetate.

9. Method of preparing a transdermal matrix system according to any one of claims 1 to 8, said method being **characterized in that** it comprises the steps wherein:
(α) the diethyl phthalate, the N-alkyl-2-pyrrolidone, the 2-octyldodecyl myristate, at least one hormone selected from the group consisting of estrogen components, progestin components and mixtures thereof, the VA/VP copolymer, if present in the formulation, and the EVA are successively introduced into a reactor at a temperature below the boiling point of the solvent or solvent system used, and the mixture obtained is stirred;
(β) the solvent or solvent system is then incorporated and the whole is stirred, still at the same temperature, until the EVA has totally dissolved and said mixture has become completely homogeneous;
(γ) the homogeneous mixture resulting from step (β) is coated onto a non-stick temporary carrier, at a temperature of between 50 and 70°C, to give a deposit of 50 to 300 g/m² on said carrier;
(δ) the coating obtained is heated to a temperature of between 40 and 80°C, depending on the boiling point of the solvent or solvent system, in order to evaporate the latter; and
(ε) the resulting dry matrix is transferred to a final carrier.

10. Use of a transdermal matrix system according to any one of claims 1 to 8 for preparing a drug intended for the treatment of the symptoms of menopause or osteoporosis.

11. A system according to claim 2 wherein the matrix comprises the following for a total of 100 parts by weight:
(a) 54 parts by weight of ethylene/vinyl acetate copolymer;
(b) 17 parts by weight of 2-octyldodecyl myristate;
(c) 5 parts by weight of diethyl phthalate;
(d) 16 parts by weight of N-dodecyl-2-pyrrolidone;
(e₁) 2 parts by weight of 17 β-estradiol;
(e₂) 5 parts by weight of norethisterone acetate; and
(f) 1 part by weight of a vinyl acetate/N-vinyl-2-pyrrolidone (VA/VP) copolymer.

12. A system according to claim 2 wherein the matrix comprises the following for a total of 100 parts by weight:
(a) 62 parts by weight of ethylene/vinyl acetate copolymer;
(b) 13 parts by weight of 2-octyldodecyl myristate;
(c) 10 parts by weight of diethyl phthalate;
(d) 10 parts by weight of N-dodecyl-2-pyrrolidone;
(e) 3 parts by weight of 17 β.estradiol; and
(f) 2 parts by weight of a vinyl acetate/N-vinyl-2-pyrrolidone (VA/VP) copolymer.

13. A system according to claim 2 wherein the matrix comprises the following for a total of 100 parts by weight:
(a) 53 parts by weight of ethylene/vinyl acetate copolymer;
(b) 17 parts by weight of 2-octyldodecyl myristate;
(c) 10 parts by weight of diethyl phthalate;
(d) 13 parts by weight of N-dodecyl-2-pyrrolidone;
(e) 6 parts by weight of norethisterone acetate; and
(f) 1 part by weight of a vinyl acetate/N-vinyl-2-pyrrolidone (VA/VP) copolymer.
